# EUROPEAN PATENT APPLICATION

(11) **EP 3 527 096 A1**
(43) Date of publication of application: **21.08.2019**
(21) Application number: 19163152.2
(22) Date of filing: 14.10.2016
(51) Int. Cl.: A42B 3/04

(54) **TEAM PARTICIPANT AWARENESS INDICATOR AND INDICATIVE NOTIFICATION**

(30) Priority: 15.10.2015 US 201514884305
(62) Divisional of application: 16787983.2
(71) Applicant: Scott Technologies, Inc., Boca Raton, Florida 33431 (US)
(72) Inventor: LERNER, Barry Michael, Waxhaw, NC North Carolina 28173 (US)
(74) Representative: Mathys & Squire

(57) **Abstract**

A remote device (70) for biometrically monitoring a subject for potential injury is disclosed. The subject wears a wearable cranial device. The remote device comprises a receiver configured to receive physiological data from at least one physiological sensor (30), impact data from at least one impact sensor (3), and environmental data from at least one environmental sensor (40), the at least one physiological sensor, the at least one impact sensor, and at least one environmental sensor associated with a wearable cranial device wearable by the subject; and a processor.

## Description

### FIELD OF THE INVENTION

The present invention relates to a device, system and method for monitoring and alerting of cranial events such as impact magnitude and location, as well as for monitoring physiological and environmental parameters.

### BACKGROUND OF THE INVENTION

Head gear is worn in a wide range of day to day activities covering both work and leisure, in particular sports. Some forms of head gear are worn primarily for protection purposes such as hard hats worn in hazardous work environments or some more assertive or dangerous sports, whilst others, such as baseball caps, are worn primarily for other purposes such as shading the head and face from the sun.

In many environments in which head gear is worn, there is a risk of head injury due to impacts with the head. The level of that risk is used to guide the type of head gear which should be worn to try to give the appropriate protection in the particular environment whilst minimizing the impact on the wearer's ability to perform a required task, play a chosen game. However, the conventional approach to head impact protection of trying to minimize the risks and effects by cushioning through head gear is limited in its usefulness because it does not allow for accountability of head trauma. Often the effects of head trauma are not immediately apparent, so that a person wearing a hard hat who is hit on the head may feel normal and therefore not seek medical attention without realizing that the magnitude of the impact may have given rise to presently unrecognized issues, for which early treatment is essential with a view to minimizing long term damage.

Systems are known in the art which overcome this problem. United States Patent no. 8,766,798 (the "'798 Patent") to Howard et al, for example, discloses a protective headgear and monitoring system in which linear acceleration and rotational velocity sensors are provided within the protective headgear which monitor movement of and impacts to the headgear and record the data for downloading to a monitoring device, upon which the data can be viewed and alarms triggered in the case that the data indicates an event has occurred which requires action or further investigation. In the system of the '798 Patent, the data is stored in the head gear for downloading at an appropriate time, and this has the drawback that it may still be some time before the severity of an event is realized, which time delay may be critical in the successful treatment of an injury.

United States Patent No. 9,041,528 (the "'528 Patent"), also to Howard et al, proposes a system in which a protective headgear uses sensors for identifying and measuring impact force which includes a transmitter which transmits data in response to an impact event at the head gear, such that an immediate alert of an event is raised. While this system offers an improvement over the system of the '798 Patent, the '528 Patent still requires a threshold trigger set for an alarm signal to be activated, that threshold being set at a level indicative of a high level impact. The '528 fails to identify a number of lower level impacts that can also give rise to injury or account for other factors affecting the well-being of person engaged in a group activity.

United States Patent No. 5,200,736 to Coombs et al discloses an assembly for monitoring helmet thermal conditions which includes a thermistor for sensing the status of an ambient thermal condition together with an alarm indicator which signals to the wearer when a number of predetermined thresholds are reached.

None of the cited references address the relational and cumulative effect of trauma, physiological and environmental factors on individual members of a group to indicate the need for timely intervention.

### OBJECTS OF THE PRESENT INVENTION

A first object of the present invention is to provide a wearable cranial device which is able to monitor a wearer's general health and identify possible injuries, and to report to a remote monitoring location. More particularly, it is an object of the present invention to detect and record impacts and transmit them to a remote monitoring apparatus, and also to collect and record physiological data from a wearer and transmit such data to a remote monitoring apparatus so that early indicators of health issues or injuries can be identified and actioned.

A still further object of the present invention is to provide a wearable cranial device which is able to collect, record and transmit to the remote monitoring apparatus environmental data.

These and other objects are met by the invention as set out hereinafter.

### SUMMARY OF THE PRESENT INVENTION

The present invention provides wearable cranial device. At least one physiological sensor is associated with the device for measuring physiological data for the individual. At least one force sensor is also integrated into the device for measuring forces associated with impacts to the exterior of the device. A transmitter is provided in the device for transmitting data from at least the at least one force sensor to a remote receiver. The device may include a substrate conformable to an individual's upper cranial hemisphere into which the sensors may be integrated.

In an alternative embodiment, the invention provides a wearable device. The device comprises at least one force sensor supported by the substrate for measuring forces associated with impacts to the exterior of the device. The at least one force sensor is configured to measure impact forces in different quadrants of the substrate, such that the data transmitted to the remote receiver enables the location as well as the magnitude of an impact to be determined. A transmitter is provided for transmitting data from said at least one physiological sensor to a remote receiver.

The present invention further provides a wearable cranial device assembly comprising a wearable cranial device and a remote display device. The wearable cranial device comprises a substrate conformable to an individual's upper cranial hemisphere, at least one physiological sensor integrated into the device for measuring physiological data for the individual, and at least one force sensor integrated into the device for measuring forces associated with impacts to the exterior of the device. A transmitter is included for transmitting data from said at least one physiological sensor and said at least one force sensor to the remote display device. The remote display device comprises a receiver for receiving data from the transmitter of the wearable cranial device, a display for displaying data from the at least one force sensor and the at least one physiological sensor, and a processor. The processor is programmed to analyze the data from the at least one force sensor and the at least one physiological sensor and identify potential injuries and/or health issues to the wearer based on the magnitude of the impact forces and/or changes in the physiological data measurements taken by the at least one physiological sensor.

Advantageously, positioning sensors, in particular global positioning systems (GPS), are also provided in the device so as to enable the location of the wearer to be ascertained from the remote receiver.

Environmental sensors are also advantageously included in the device to collect environmental parameters such as ambient temperature, humidity, solar intensity, pollen count and the like.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1A illustrates a front side view of the cranial monitoring device placed on an individual's head according to the invention;
FIG, 1B illustrates a rear side view of the cranial monitoring device placed on an individual's head according to the invention;
FIG. 2A illustrates the cranial device of the invention used in conjunction with a hard hat;
FIG. 2B illustrates the cranial device of the invention used in conjunction with a baseball cap;
FIG. 3 illustrates a representative protocol of the information transmission of the cranial device for alert to an impact event on the cranial device;
FIG. 4A illustrates a remote display device indicating the status of an individual which works in conjunction with the cranial device of Figures 1 and 2;
FIG. 4B illustrates a remote display device indicating the summary of the status of a group of individuals which works in conjunction with the cranial device of Figures 1 and 2;
FIG. 5 illustrates the inter-relationship of the impact factor, physiological factor and environmental factor on a transformative x, y, z graphical representation;
FIG. 6 illustrates a mesh network communication system for an array of cranial devices in a sports environment of the invention; and
FIG. 7 illustrates a mesh network communication system for an array of cranial devices in a work environment of the invention.

### DETAILED DESCRIPTION OF THE PREFERRED EMBODIMENT

The present invention is a device, system and method for accounting for the relational and cumulative effect of trauma, physiological and environmental factors on individual members of a group to indicate the need for timely intervention. Proper accounting for the relational effect of these factors allows for the proper assessment by monitors, such as supervisors, coaches or parents, to receive and identify problematic trends or circumstances where individuals of a group are likely to be physically compromised. The invention includes a cranially worn device which includes sensors for registering an individual's head trauma, physiological indicators, and environmental conditions. With the collection of this data the well-being of the wearer is accessed, monitored and reported to a remote location.

There are a wide range of activities undertaking by a human, both for work and for leisure purposes, during which they can be exposed to environmental factors which can impact on their health and well-being. Those environmental factors can be ambient temperature, humidity, sun exposure and the like which can cause health issues which as sunstroke, sunburn, dehydration and the like, foreign object impact such as from falling debris, impact from a projectile or the like which can cause cranial injury such as concussion. A sports player playing a game such as American football or baseball can often spent long periods of time out on the field during which time the environmental factors can give rise to health issues without the person knowing. Dehydration is often unnoticed by the suffering until secondary symptoms arise, and the same is true of sunstroke and exposure. Equally, the magnitude of a cranial impact such as caused by being struck by a hit ball in baseball or through impact with another player in football is important data for correct identification of the significance of the event and whether medical intervention is required. Early identification of extreme trauma can be critical in successful treatment.

In a preferred predictive embodiment, the invention calibrates these diverse factors to formulate proper factor tolerances for early notification. Although a severe event of any one of the factors on its own may create a critically significant health hazard notification of the individual, less dramatic changes in any of these factors redefines the acceptable boundaries of the other factors, as the individual becomes less capable to withstand change in the other factors. As the effect of any one of these factors compromises an individual's ability to withstand the effects of the other factors the indicator limits for the factors migrate to new levels.

In a preferred indicative embodiment, the invention evidences the significance of an occurrence of the impact factor, and provides a confirmatory measurement. In this embodiment as an impact occurrence happens, changes in the physiological factor, which may be in combination with the environmental factor, evidence the severity of the impact. As changes in the physiological factors happen as a result of the cranial impact to the individual, this provides additional data for analytical consideration for the determining the severity of the impact to the individual. As such an otherwise seemingly moderate impact may be recalculated as a significant impact with the changes in the physiological factors registered post-impact.

The present invention therefore provides a system for remote monitoring of a subject for cranial impacts as well as for physiological changes to enable remote and early identification of diminished performance, possible injury or health issues.

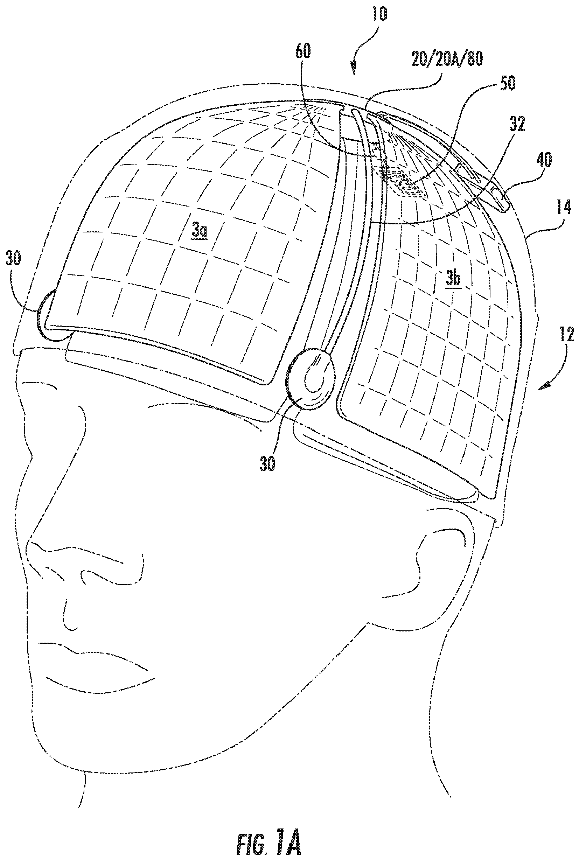

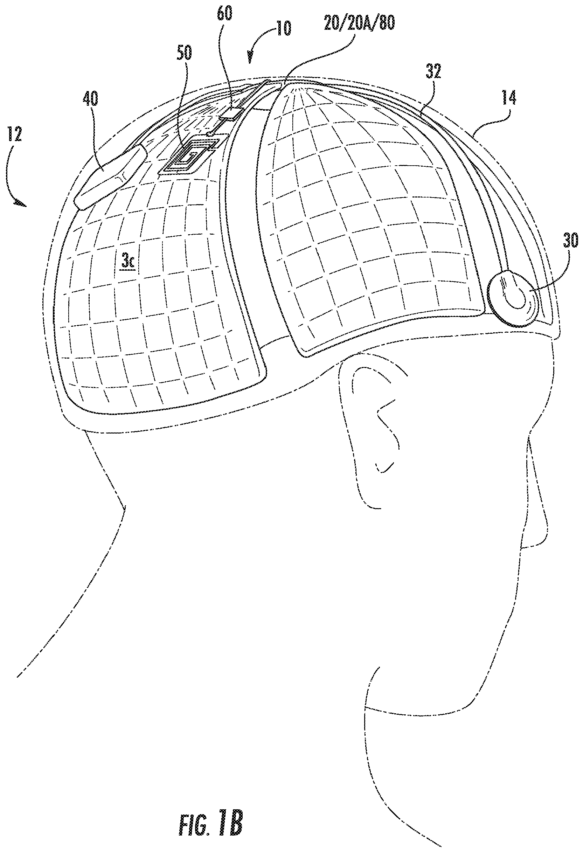

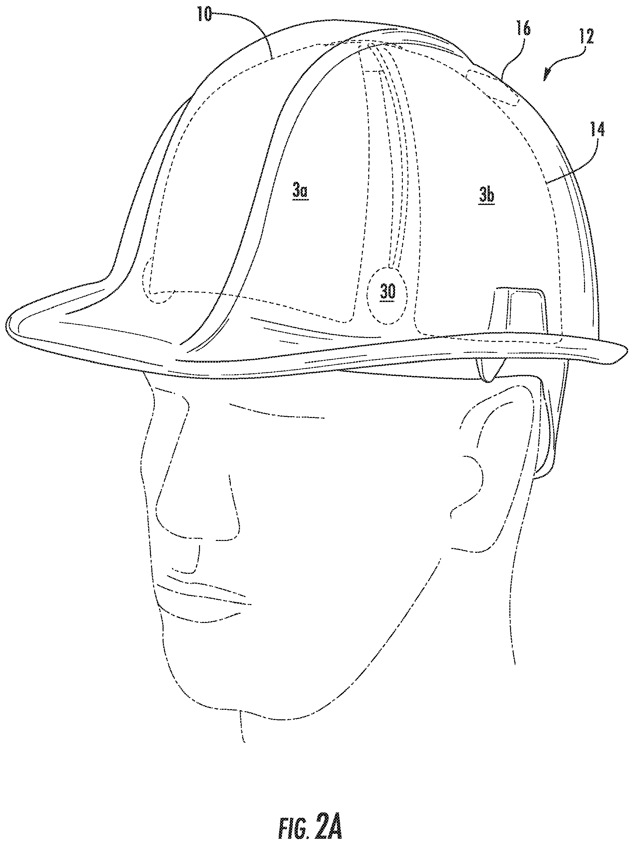

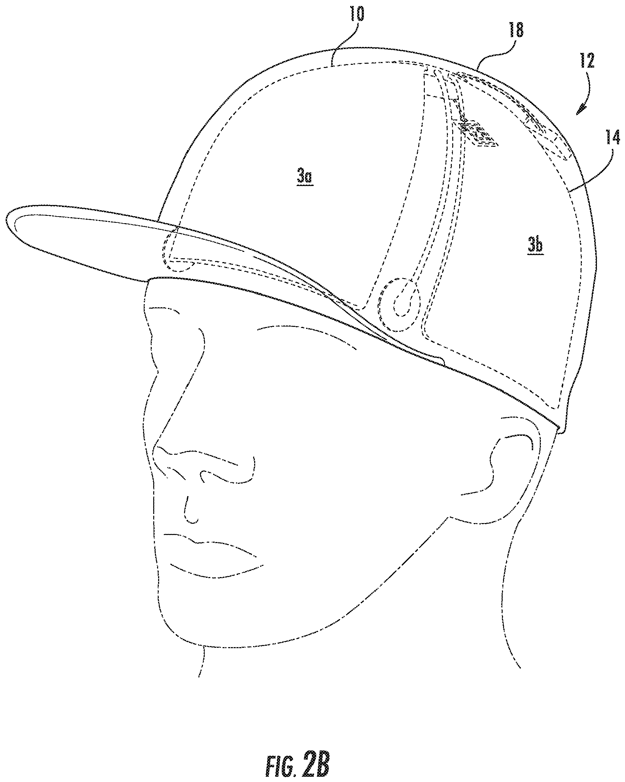

Referring first to Figures 1A and 1B, there are shown exemplary representations of a cranial monitoring device 10 according to the invention. The illustrated example of Figure 1A shows a front side view of the cranial device 10 realized as a stand-alone product which is worn on the upper portion of the wearer's head 12. The illustrated example of Figure 1B shows a rear side view of the same cranial device 10. As seen in Figures 2A and 2B, the cranial device 10 can be worn, for example, under another piece of headwear such as a work helmet 16 as shown in Figure 2A, or sports hat 18 as shown in Figure 2B. One of ordinary skill in the art will recognize that the invention may also be integrated into other types of headwear for the purposes of the invention described herein.

Referring to Figures 1A and 1B, the cranial device 10 has a supporting substrate 14, which substrate 14 is shaped to conform to the shape of a wearer's head 12 and thereby provide a close fit to the wearer's head 12. This allows certain sensors incorporated into the cranial device 10 to remain in close proximity, and preferably in contact, with the wearer's head 12 in order to gather relevant and accurate data. The substrate 14 can be formed of an elastomeric material to facilitate a close fit or be molded to the shape of a specific wearer's head 12. The substrate 14 preferably includes a cushioning material between the cranial device 10 and wearer's head 12 for comfort and fit.

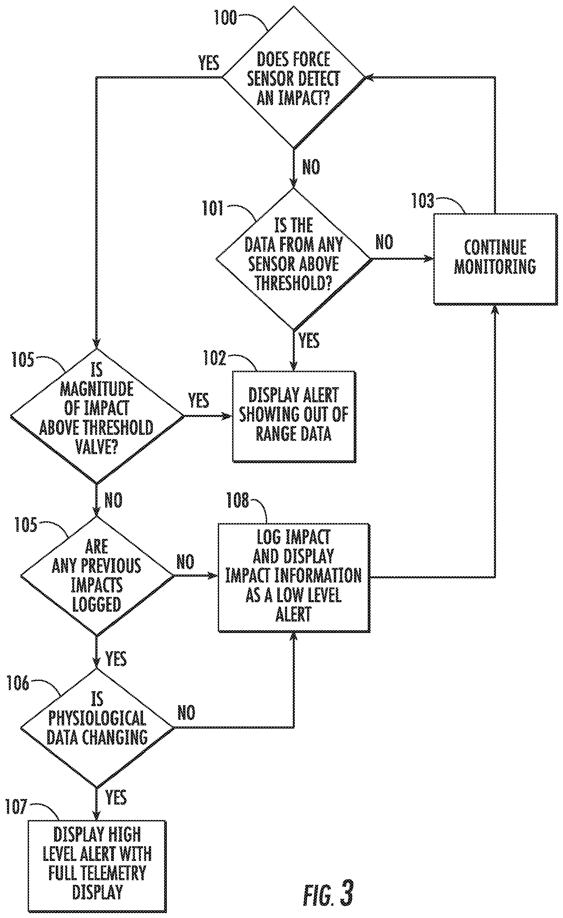

The cranial device 10 includes a number of impact sensors 3a, 3b, 3c and 3d, generally positioned by, and preferably fastened to, the substrate 14. The impact sensors 3a-3d detect impact force on areas of the substrate 14. Preferably the location of the impact sensors 3a-3d is on the inside of the substrate 14 to ensures the impact sensors 3a-3d each measure accurately the actual force imparted to the wearer's head 12 prior to the dampening effects of any cushioning included between the impact sensors 3a-3d and the wearer's head 12 as provided by the substrate 14 as a standalone device as shown in Figures 1A-1B, or as worn over or incorporated within a protective helmet or other head device as shown in Figures 2 and 3. The impact sensors 3a-3d are distributed throughout the substrate 14 by region or other identifiable areas of the wearer's head 12 so as to detect impacts in different regions of the cranial device 10 associated with different quadrant's of the wearer's head 12. In this way, as described hereinafter, the system can detect not only the occurrence and magnitude of an impact, but also the location of that impact, the accuracy of the location information being dependent on the number and distribution of the impact sensors 3a-3d. The cranial device 10 can include a sensor to detect being located on an individual's head. In one embodiment the cranial device 10 includes a single impact sensor 3a that covers the entire head or one or more specified regions of the wearer's head 12, e.g., having a neural network to identify specific locations of impact over the entire area of the head covering, and preferably the cranial device 10 includes a plurality of impact sensors to further define the located of an impact on the wearer's head 12, with one preferred embodiment having four impact sensors 3a, 3b, 3c, 3d provided covering the front left, front right, rear left and rear right quadrants of the wearer's head 12. As more accurate locating of impacts is required, more impact sensors 3a-3d are used with the distribution and size of each impact sensor 12 being determinable by one skilled in the art for a particular use of the cranial device 10, such as a work usage, sports usages, etc. Impact sensors 12 can be combined into a single piece of headwear using differing sizes and constructions of the impact sensors 12.

Each impact sensor 3a-3d is physically connected to a central module 20, or otherwise, for communicating the data from each impact sensor 3a-3d to the central module 20 for further processing as described hereinafter.

The cranial device 10 also includes, or can be used in combination with, one or more physiological sensors 30 which can be mounted to or separated from the inner surface of the substrate 14 so as to engage with the wearer's head 12 and gather physiological data from the wearer. The physiological sensors 30 collect data from the wearer relating to the wearer's physiological factors, such as without limitation, core temperature, heart rate, perspiration, and blood pressure. Each physiological sensor 30 is physically connected to the central module 20, or otherwise such as by connector cables 32 which transmit the data from each sensor 30 to the central module 20. One preferred embodiment includes the physiological sensors 30 housed within the cranial device 10. In a second preferred embodiment the physiological sensors 30 are integrated in ancillary personal attire, such as for example without limitation, shirts, wristbands, chest straps, headbands, socks, shoes, watches and the like, and combinations thereof, with such devices being physically wired or wirelessly connected or otherwise electronically configured to combine with the cranial impact data from the cranial device 10. For example, cranial device may form part of a general sensor array which also includes ancillary devices for physiological monitoring, each device communicating with a central processor also carried or worn on the body of the wear which, in turn, transmits data from all of the sensors to a remote monitoring location.

The cranial device 10 also includes, or can be used in combination with, environmental sensors 40 which take localized environmental readings relating to the atmosphere immediately surrounding the cranial device 10. The environmental sensors 40 are typically located on an exterior surface of the substrate 40 so as to isolate the environmental sensors 40, as much as possible, from the effects of the wearer's physiological temperature of the wearer his or herself, for example isolating the impact of an increased temperature readings due to the effects of expired air. The environmental sensor 40 is preferably impact resistant and/or has additional cushioning to protect it against external impact. Preferably the environmental sensor 40 is configured to not attenuate any impact event onto the wearer such as by having smooth, padded edges and the like. The environmental sensors 40 are configured to collect data of one or more of ambient temperature, ambient humidity, ambient air pressure, ambient light level, solar intensity, and the like. The environment sensor 40 can also be configured to measure the temperature above the top of the cranial device 10, corresponding with the temperature above the wearer's head 12 and inside the helmet 16 or other headwear 16, 18 worn over or integrated with the cranial device 10. Each environmental sensor 40 is connected to the central module 20 by a connecting means for data transmission from each environmental sensor 40 to the central module 204.

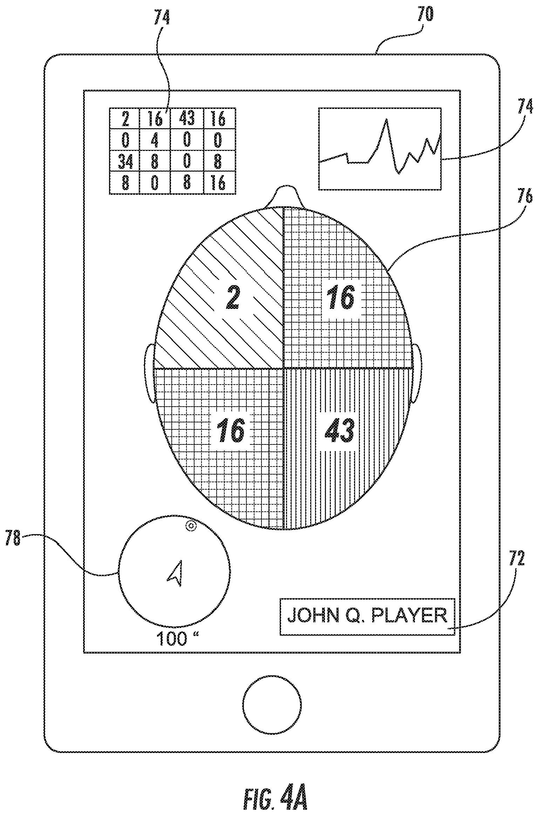

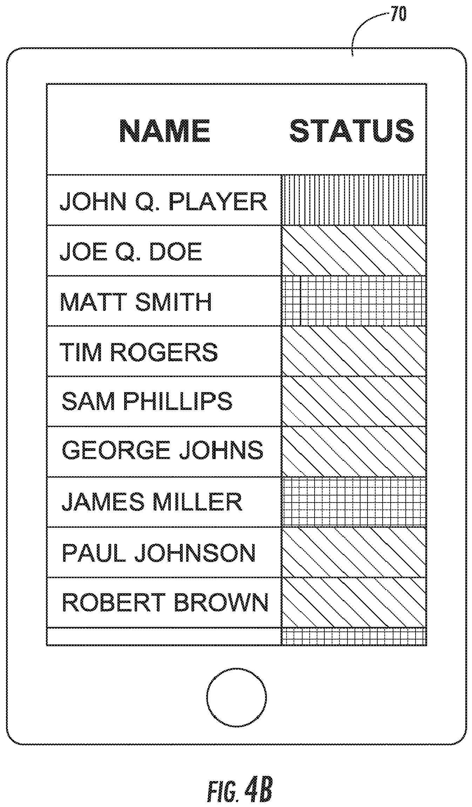

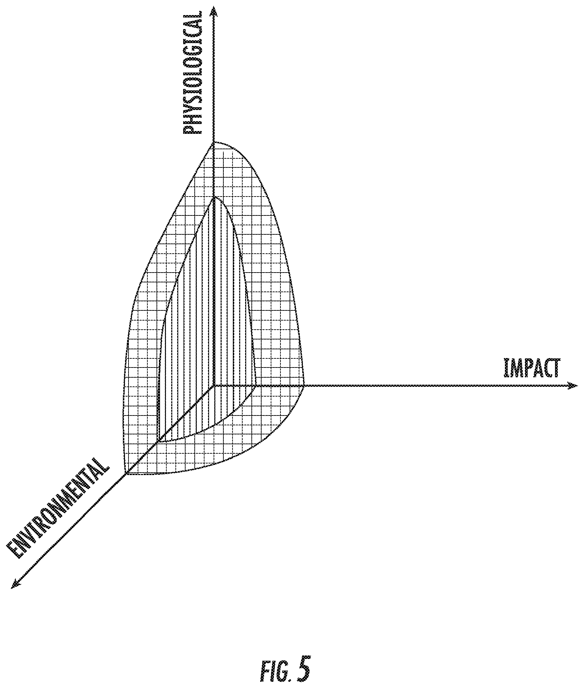

The central module 20 is configured to receive data from each of the impact 3a-3d, physiological 30 and environmental 40 sensors, and preferably includes a storage module 20A for storing the data from each sensor. The central module 20 also includes a transmitter 50 for wirelessly transmitting the data collected from each sensor to a remote receiver 70 (shown in Figures 4 and 5). The transmitter 50 transmits the data using suitable transmission systems such as Bluetooth, Wifi, RF transmission or the like. A power source 60 is included in the module for powering each of the sensors, as needed, the transmitter 50 and storage module 20A, and a suitable antenna may be integrated into the storage module 20 or into the substrate 14 for maximizing the range of the transmitter 50. The storage module 20 also includes an ID tag which is unique to the particular cranial device 10 so as to enable the cranial device 10 to be uniquely identified by the remote receiver 70 and the data received uniquely associated with the wearer of the cranial device 10. This is important since, as described below, the remote receiver 70 is preferably configured to receive and process data from a number of different cranial devices 10. As indicated above in a second preferred embodiment, the central module 20 may be located separately from the cranial device and receive and process data from a number of separate sensors worn on the body of the user.

The device 10 also includes a location sensor 80 such as a global position system (GPS) receiver by means of which the location of the cranial device 10 is identified and transmitted to the remote receiver 70 so that the location of the wearer can be ascertained at any time from the remote receiver 70 as described hereinafter. In the illustrated embodiment, a GPS receiver is integrated into the central module 20 with antenna integrated into one or more of the wires connectors connecting the central module 20 to the sensors. However, the GPS receiver or other location module may be separate from the central module 20 and connected thereto for transmitting the location data to the remote receiver 70.

As illustrated in Figures 2A and 2B, in the preferred embodiment the cranial device 10 is sized so as to be wearable as an under-layer beneath other types of head attire, such as under, or integral to, a baseball cap (Figure 2A), a sports helmet, a workman's hard hat (Figure 2B), including for example without limitation, hockey helmet, bicycle helmet, motorcycle helmet, and the like. This list is not intended to be exhaustive but instead to provide a small number of examples of the type of head wear with which the cranial device 10 may be worn.

An example of the monitoring of a wearer will now be described in conjunction with the flow chart of Figure 3. The remote display 70 continuously monitors the telemetry received from the cranial device 10 of the user. An impact to the wearer's head will be a sudden event, whereas changes from the other sensors will typically be a gradual change. If no impact is detected, the system tracks the other sensor output for changes and in particular for changes which result in a parameter exceeding a preset threshold value. If that is detected at 101, an alert is immediately display 102 and/or sounded, with the display showing all relevant data as well as the data for the out of range parameter. For example, if the telemetry indicates that heart rate has slowed and ambient temperature is elevated, although still below threshold, this will be displayed together with a warning of possible dehydration 102. Otherwise, monitoring is continued 103.

If an impact is detected at 100, the system first checks if the level of the impact exceeds the threshold level 104. If it is does, an alert is immediately displayed showing full telemetry for the wearer 102. If the threshold is not exceeded, a check is made to see if there have been any previous impacts logged 105. If there have not, then a low level alert is displayed showing the area and magnitude of the impact, and the occurrence, location and magnitude of the impact is recorded for future reference 108. Monitoring is continued with secondary, more stringent threshold values for physiological data being applied for the wearer.

If previous impacts are recorded, the display immediate activates a baseline for all physiological data based on the last readings prior to the impact and starts monitoring for any changes from the base line physiological readings 106. The further impact, including its location and magnitude is recorded for the wearer and the active readings display with a mid-level alert. If any changes are detected to the physiological data, a high level alert is immediately displayed showing all telemetry for the wearer 107.

The example shown in Figure 3 represents monitoring of a particular event and it will be understood that the invention is not limited to the particular logic applied in the flow chart of Figure 3.

Referring next to Figures 4A and 4B, there is shown a schematic representation of a remote receiver 70 which operates on conjunction with the cranial device 10, receiving data therefrom by means of the transmitter 50 and displaying the information to a remote user from the wearer.

Referring to Figures 4A and 4B, more particularly, the remote receiver 15 has a receiver which receives data transmitted by the transmitter 50 in a cranial device 10, either directly or indirectly. The remote receiver 70 includes a processor which is programmed with threshold "event" values for each of the impact, physiological and environmental parameters collected and transmitted by the cranial device 10 such that, upon one or more of the data readings transmitted from the cranial device 10 exceeding the pre-programmed threshold value, an alert 72 is signaled on the remote receiver 70 in the form of a visual alert of the screen and/or an audible alarm, the visual alert identifying the cranial device 10 and/or wearer which has given rise to the alert as well as displaying the or each parameter 74 which has exceeded the threshold. In the case of, for example, a cranial impact which exceeds the threshold level, the remote receiver 70 is advantageously programmed to display a 2 dimensional or 3 dimensional presentation of a human head 76, indicating the area of the impact as well as the severity shown by quadrant in Figure 4. The remote receiver 70 may also be programmed to display potential identify possible injuries which may have been incurred by the wearer of the device 10 as a result of the parameter or parameters which have activated the alarm. Additionally the remote receiver 70 can identify the position 78 of the wearer of the cranial device 10, such as by a direction and distance or plotting the location of the cranial device 10 and remote receiver 70 on a represented map displayable on the remote receiver 70.

The remote receiver 70 is further programmed to correlate the data from all of the sensors in the cranial device 10 together to identify possible health and well-being issues either arising from alarm triggering events or not. For example, upon an impact to the cranial device 10 triggering an alarm due to being above the pre-programmed threshold value, the remote receiver 70 may automatically display all telemetry 74 from the cranial device 70 and highlight any changes to other data, whether or not those changes are themselves alarm triggering changes. The remote receiver 70 will further identify that, for example, following a head trauma the blood pressure of the wearer has started to drop or the heart rate, and identify possible injuries which may have therefore been incurred as well as the severity and recommended action to take. Triggering parameters can be designed from standard health schedules, such as standard parameters for the general population, or males ages 14-18, or mixed sex populations of 34-65, and other such sub-divided population groups, or can be developed per individual using historical parameters of that individual.

The remote receiver 70 is still further programmed to store and analyze the data from the cranial device 10 and identify possible issues arising from changes or events which occur over time. For example, the wearer may have been subjected to a number of cranial impacts, each of which on its own does not exceed the threshold valve but do to the number of blows received and the time frame in which they have been incurred, the remote receiver 70 may nonetheless trigger an alarm or warning for the wearer of the cranial device 10.

Similarly, the remote receiver 70 will correlate physiological data with environmental data, so that if, for example, the ambient temperature has exceeded a certainly level after which physiological parameters start to change, even if those changes are not outside allowable values, the programming of the remote receiver 70 will reset or override the parameter limits and identify possible injuries or health issues with the wearer indicated by the readings from the various sensors in the cranial device 10.

As seen in Figure 4B, the remote receiver 70 can include an indexing function for accounting for the status of a group or team. This indexing screen allows an overview that preferably has an immediate conversion to review individuals, as shown in Figure 4A, by tapping on an individual's name. Additional data analysis includes comparison of an individual's parameters with those of his/her team members, preferably in analytical terms such as without limitation, mean average or standard deviation, with and without reference to comparisons of standard and individualized parameter limitations.

It will be understood that the above are examples of the types of analysis and alerts which the system of the invention enables. By providing sensors for a number of different parameters, the cranial device 10 is able to support general health and well-being of the wearer by not only monitoring the data individually by also correlating the data received from the numerous sensors and also by tracking changes in the data over time. In this way, indication of problems and also prediction of possible upcoming problems like dehydration, sun burn etc. can be made and preventative action taken.

The remote receiver 70 furthermore optionally includes a data store for storing personal profile for the wearer of a cranial device 10 which is currently being monitored. This may include, but be limited to, age, weight, height, sex and relevant medical history, including previous injuries. This personal information may be updated by the remote receiver 70 with current telemetry to record, for example, a head injury which the severity and location to be taken into account in future analysis of telemetry data for that wearer. The personal information may also include personalized values for the threshold values at which alerts are triggered for the different monitored parameters.

The remote receiver 70 also receives and analysis the location data transmitted by the or each cranial device 10 with which it is in communication. The remote receiver 70 is then able to display to the user the location 78 of any or all cranial devices 10 which it is monitoring. The remote receiver 70 may be programmed with a number of different display options. For example, it may be possible to provide a display which shows the location of all active cranial devices 10 on a map type layout. This may include indicators to highlight the location of any wearer who has been identified as having possible injury or well-being issues. For example, icons may change color from, say, green to red when readings are detected which have been identified as being indicative of possible problems. A representation 76 of a human brain may also be displayed, showing the different monitored areas and showing the level of any impacts detected thereto - both current and historical. This may be a 2D display as shown in Figure 2 or a 3D display The remote receiver 70 may also include a location option 78 in which a compass type display is provided with an arrow to indicate a direction towards a selected cranial device. This would be particularly useful in an environment where monitored cranial devices 10 are out of visual range. In that case, if an event such as a head trauma is detected, the remote receiver, having detected and alerted of the impact, can then be used to easily find the wearer. A two way communication system may also be included to allow voice communication between the remote receiver 70 and a cranial device 10 wearer, enabling conversation to check the status of the wearer and/or provide comfort whilst help is dispatched.

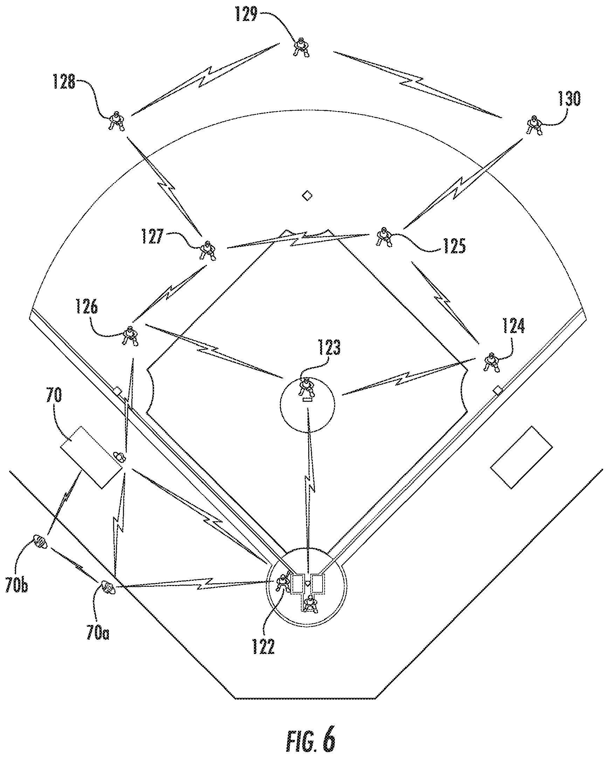

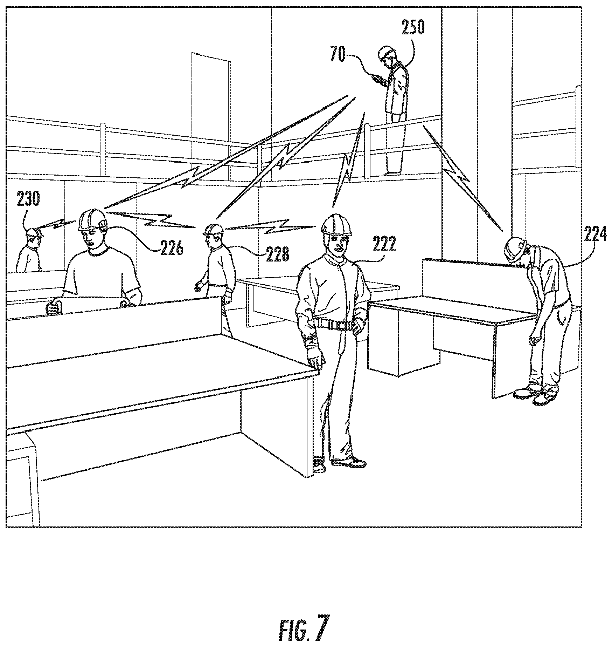

Primary communication between the cranial device 10 and the remote receiver 70 is by a direct communication link. However, in a preferred embodiment, each cranial device 10 includes a receiver, in the embodiment illustrated in Figures 1 and 2 which is integrated with the transmitter into the central module 20. In addition to the receiver enabling two way communication to take place between the remote receiver 70 and the cranial device 10, as seen in Figures 6 and 7, the receiver in the cranial device 10 is able to receive and re-transmit data transmitted by other cranial devices within its network in the manner of a relay station. This mesh network arrangement enables the communication range of the cranial devices 10 to be extended as illustrated in Figure 6 and 7.

Figure 5 represents layers of alarm for set parameters of impact, physiological, and environmental factors that affect an individual during work or play. As seen in Figure 5 the defined regions represent demarcations of calculated conditions of the individual for the combinations of impact event, physiological event, and environmental event on an individual. In one embodiment, the center point x, y, z = 0, 0, 0 represents no impact event, a baseline physiological event, and a baseline environmental event. Moving along the positive x-axis, impact events are registered. For example an impact event in the vertical lined region along the x-axis may indicate an impact on a junior league baseball player who is slapped on his hat by a teammate using a glove. Moving further down the x-axis, the region filled with squares may represent a more forceful impact such as the hat hitting the ground while sliding into second base. The area outside of these defined regions would represent a significant impact, such as a direct ball hit to the hat. Likewise the physiological y-axis and environmental z-axis provide defined regions of factors that affect the individual with increasing levels of concern, and can be defined by data ranges, data limits, specific values, or the like. The regions are determined by one of ordinary skill in the art for specific uses, such as youth baseball, industrial workers in strenuous activities, etc. In intersection of the degree of effect on the well-being of the individual varies by age, sex, conditioning, and other like factors of the individual and the limits desired to account for the individual's status, such as the individual's history. For example, the limits may be varied in real-time to account for an impact for determining the limits of the next impact. Additionally the limits can be determinative of predictive and/or indicative analysis.

Figure 6 shows an example of the invention being used to monitor players in a baseball game, only players 122 and 126 are within direct range of the remote display 70. However, the remote display 70 is able to receive data from all players on the field through the mesh network communication system. Player 129, who is furthest from the remote display 70, is, nonetheless within range of left and right fielders 128, 130 and data transmitted by cranial device 129 is received by each of cranial devices 128 and 130. Device 128 which is also out of range of the remote display 70, will transmitted both its own telemetry data as well as that received from device 129. This will be received by cranial device 127 which will, in turn, transmit that data onwards with its own telemetry data. Cranial device 127 will also receive data from device 125, which will include telemetry received from devices 129, 130 and 124. This relaying of telemetry data will continue until the telemetry is received at either of the cranial devices 122, 126 which are within range of the remote receiver 70, at which point the data is transmitted to the remote display 70 and processed. Each cranial device will have a unique identifier which will be encoded in the telemetry transmission so that the remote display 70 can decode the data and identify which data has come from which device. Each device also embeds a time stamp with the data transmission so that duplicated data received along a different route through the mesh network can be identified by the remote display 70.

As described above, in the illustrated embodiment, the remote display 70 works with a number of cranial devices configured in a network, enabling the monitor to track the well-being of all the players under his or her supervision. However, each device may instead be configured to communication with just a single remote display 70 which may, for example, be controlled by the parent of the wearer of the device. In that case, communication range may still be extended using the mesh network configuration described above, each remote display 70 only being able to decode and display data received from its paired cranial device and merely acting as a relay within the mesh network for data received from any other cranial device.

In a particularly preferred configuration, a master remote display 70 is configured within the network which is able to receive, decode and display data from all cranial devices within the network. Secondary display devices 70a, 70b are also able to receive data but each secondary display 70a, 70b is only able to decode and display data coming from its paired cranial device or devices. For example secondary display 70a may be controlled by the parent of player 125 and secondary display 70b may be controlled by the parent of players 126 and 129. Display 70a will receive data from all cranial devices in the mesh network but will only decode and display data coming from the child's device 125, whereas display 70b is able to decode and display data from the cranial device worn by player 126 and player 129. Master display 70, on the other hand, receives and displays data from all players.

The master display 70 may be configured to act as a relay to secondary displays so that data transmission to the secondary displays 70a, 70b is controlled and restricted by the master display 70, the master display, for example, filtering data which is transmitted onwards. Even in this configuration, secondary displays 70a, 70b may still act as replay stations within the mesh network.

Whilst particular examples set out above are in relation to use of the invention in connection with leisure activities, it will be understood that the invention is equally applicable in work environments, the invention enabling tracking of personal and monitoring of their well-being. For example, the invention may be integrated into fire-fighter's helmets so that health and well-being of each fire fighter in a hazardous environment can be monitored in real time. In that application, environmental sensors may additional monitor oxygen / carbon monoxide levels in the environment and also monitor for other hazardous or toxic substances.

Additionally Figure 7 shows an example of the invention being used to monitor workers in a facility or at a location where workers 222, 224, 226 and 228 are within direct range of the remote supervisor 250 holding display 70. However, the remote display 70 is able to receive data from all workers in the work area through the mesh network communication system. Worker 230, who is furthest from the remote display 70, is, nonetheless within range of worker 226 and data transmitted by cranial device 230 is received by the cranial device 226. Device 226 which is in range of the remote display 70, will transmit its own telemetry data as well as that received from device 230. This relaying of telemetry data will continue as the telemetry data continue to establish links with other units that are in range to transfer data to the remote receiver 70, at which point the data is transmitted to the remote display 70 and processed. Each cranial device will have a unique identifier which will be encoded in the telemetry transmission so that the remote display 70 can decode the data and identify which data has come from which device. Each device also embeds a time stamp with the data transmission so that duplicated data received along a different route through the mesh network can be identified by the remote display 70.

The display device 70, as well as secondary display devices 70a, 70b, may be equipped with a transmission system such as a GSM communication system and the like by which data may be transmitted from the display to a remote site, such as a hospital to assist medical practitioners with treatment of injuries and health issues detected by the display.

The remote display may be a dedicated device supplied with the cranial device 10. However, the remote display is preferably realized by an application (App) for a smart phone which is written to work in conjunction with the cranial device 10. The App may be configured with primary functions which are enabled in a basic version of the App as well as premium functions which are unlocked through in App purchases.

Additionally, the invention may combine or integrate data and systems that monitor an individual's well-being, such as health monitoring through sensors in clothes, wristbands or footwear. Other integrated data may include measurement of altitude, pollen, smog, and other like local environmental conditions that can affect an individual's response to head trauma.

In order to further illustrate the applications and advantages of the invention, there will now be described two examples of the practical application and use of the invention, one relating to use in a leisure application and one in a work place application.

### Example 1 - Monitoring of a little league baseball player

With reference again to Figure 6, 9 year old Mike 125 is fielding close to second base in his little league game, watched by his dad 70b. The entire team are equipped with cranial monitoring devices according to the invention and the coach 70 has the primary remote receiver 70 which monitors data from the entire team. Mike's dad also has the monitoring app downloaded onto his smart phone so that he can monitor Mike's cranial device 125 as a secondary remote receiver 70b.

The batter 122 hits the ball towards Mike 125 who is hit on his frontal lobe. The front impact sensor 3a detects and measures the impact and transmits the data back to the remote receivers 70, 70b through the mesh via cranial devices 127, 126. The primary 70 and secondary 70b remote devices both immediately display the impact even, showing the location and magnitude of the impact and also indicating that, in this case, it is below the threshold level, although only just below so an amber warning is displayed. Mike is within visual range and appears to be fine, signaling that he is OK. The data from the other sensors is not indicating any physiological problem so the game continues. Each of the remote display devices logs the impact event.

After about 5 minutes, the physiological sensors start to detect changes in physiological data from Mike. Although the changes in this physiological data is within normal threshold valves, the previous impact event logged by the remote displays 70, 70b causes each of the remote displays to immediately display a high level alert for Mike, displaying the changing physiological data and also re-displaying the data for the previous cranial impact. Both the coach and Mike's dad respond to the alert, stopping the game and providing Mike immediate medical attention.

### Example 2 - Monitoring of Workers in an industrial site.

Joe works in a factory where he is equipped with a cranial device 230 of the invention for wearing under his hard hat. His device 230, as well as those of the others in his team, is linked to a remote display 70 carried by his supervisor 250.

Joe leaves the supervised factory area to go to the store room to collect some parts and while there, a box falls from a shelf and hits him on the head, knocking him unconscious. Joe also has a known heart condition and the impact triggers a heart attack.

Data from Joe's cranial device is transmitted via the mesh network to the remote display 70 held by the supervisor 250 which immediately displays an alert both for the significant cranial impact, displaying both the magnitude and the location of the impact, as well as the out of range physiological data resulting from the heart attack. Joe's medical information is programmed into the remote display 70 and the possibility of a heart attack is also displayed on the display 70. Joe is not in visual range of the supervisor 250 but the supervisor is able to use data from the location sensors in Joe's cranial device 230 to pinpoint his location in the store room. The supervisor 250 immediately summons medical assistance using the remote display 70, transmitting the impact and physiological data to the medical team whilst going directly to the store room himself to provide immediate assistance.

From the above description and examples, it will clearly therefore be understood that the system of the present invention provides a solution for monitoring, recording and maintaining accountability and the well-being of people in a manner which is far more comprehensive than has been previously known in the art, the range of information monitored by the cranial device 10 enabling much more comprehensive analysis and diagnosis of issues to be performed by correlating information from the different sensors than has been possible with prior art devices.

While certain embodiments of the disclosure have been described herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

### Aspects of the invention:

1. A wearable cranial device comprising:
   at least one force sensor supported by the device for measuring forces associated with impacts to the exterior of the device;
   at least one physiological sensor associated with the device for measuring physiological data for the individual; and
   a transmitter for transmitting data from said at least one force sensor to a remote receiver.
2. A wearable device according to aspect 1, wherein the device further includes a substrate conformable to an individual's upper cranial hemisphere.
3. A wearable cranial device according to aspect 2, wherein the at least one force sensor is integrated into the substrate so as to measure impact forces transmitted to the head of the wearer.
4. A wearable cranial device according to aspect 2, wherein the at least one physiological sensor is integrated into the substrate.
5. A wearable cranial device according to aspect 1, wherein the at least one force sensor is configured to measure impact forces in different quadrants of the substrate, wherein the data transmitted to the remote receiver enables the location and the magnitude of an impact to be determined.
6. A wearable cranial device according to aspect 1, wherein the at least one physiological sensor measures physiological data selected from the list comprising temperature, blood pressure, heart rate, perspiration, and combinations thereof.
7. A wearable cranial device according to aspect 1, wherein the at least one force sensor measures linear impact forces and torsional impact forces.
8. A wearable cranial device according to aspect 1, wherein at least one environmental sensor is included in the device for measuring at least one environmental parameter.
9. A wearable cranial device according to aspect 8, wherein the at least one environmental parameter is selected from the list comprising ambient temperature, ambient humidity, ambient air pressure, solar intensity and ambient light level.
10. A wearable cranial device according to aspect 1, further including a location sensor for identifying the location of the device, the transmitter being configured to transmit the location to the remote receiver for enabling remote locating of the wearer of the device.
11. A wearable cranial device, comprising:
   at least one physiological sensor integrated into the device, the sensor measuring bioeffecting parameters pertaining to an individual's physical state;
   at least a second sensor including an impact force indicator supported by the device, said impact force sensor configured to measure impact forces in defined sectionalized areas of the substrate;
   a transmitter for transmitting data from the at least one physiological sensor and the at least one second sensor to a remote receiver; and
   a power source.
12. A wearable cranial device according to aspect 11, further including a substrate comfortable to an individual's upper cranial hemisphere.
13. A wearable cranial device according to aspect 11, wherein the at least one physiological sensor measures physiological data selected from the list comprising temperature, blood pressure, heart rate, and pupil dilation.
14. A wearable cranial device according to aspect 11, wherein the at least one force sensor measures linear impact forces and torsional impact forces.
15. A wearable cranial device according to aspect 11, wherein at least one environmental sensor is included in the device for measuring at least one environmental parameter.
16. A wearable cranial device according to aspect 15, wherein the at least one environmental parameter is selected from the list comprising ambient temperature, ambient humidity, ambient air pressure, solar intensity and ambient light level.
17. A wearable cranial device according to aspect 11, further including a location sensor for identifying the location of the device, the transmitter being configured to transmit the location to the remote receiver for enabling remote locating of the wearer of the device.
18. A wearable cranial device assembly comprising a wearable cranial device and a remote display device, wherein:
   the wearable cranial device comprises:
      at least one physiological sensor integrated into the device for measuring physiological data for the individual;
      at least one force sensor integrated into the device for measuring forces associated with impacts to the exterior of the device; and
      a transmitter for transmitting data from said at least one physiological sensor and said at least one force sensor to the remote display device;
   the remote display device comprises:
      a receiver for receiving data from the transmitter of the wearable cranial device;
      a display for displaying data from the at least one force sensor and the at least one physiological sensor;
      a processor programmed to analyze the data from the at least one force sensor and the at least one physiological sensor and identify potential injuries and/or health issues to the wearer based on the magnitude of the impact forces and/or changes in the physiological data measurements taken by the at least one physiological sensor.
19. A wearable cranial device according to aspect 18, further including a substrate conformable to an individual's upper cranial hemisphere.
20. A wearable cranial device assembly according to aspect 18, wherein the cranial device includes defined sectionalized areas, the at least one force sensor measuring the magnitude of an impact force and the area in which it has occurred and transmitting both the magnitude and the location data to the remote display device.
21. A wearable cranial device assembly according to aspect 20, wherein the display device provides a graphical representation of the location and magnitude of an impact on the head of the wearer using the magnitude and location data from the at least one force sensor.
22. A wearable cranial device assembly according to aspect 18, wherein the remote display device further comprises a data store for storing general user data, historical health data and historical physiological data relating to the wearer of the cranial device, the processor being further programmed to utilize said wearer data diagnose possible injuries incurred by the wearer based on the data from the at least one force sensor and the at least one physiological sensor.
23. A wearable cranial device assembly according to aspect 22, wherein the general user data, historical health data and historical physiological data comprises at least one of the age of the wearer, the sex of the wearer, known health issues of the wearer, disabilities of the wearer, previous injuries of the wear, the weight of the wearer and the height of the wearer.
24. A wearable cranial device assembly according to aspect 18, wherein the remote display device includes an alarm for alerting a user to the possible existence of and the potential severity of an injury.
25. A wearable cranial device assembly according to aspect 18, wherein the cranial device is uniquely paired with the display device so that only the display device can receive and display data from said cranial device.
26. A wearable cranial device assembly according to aspect 18, wherein the display device is associated with a plurality of cranial devices so as to receive simultaneously data from said plurality of cranial devices, the display of said display device being configured with a plurality of display modes to enable the user to data from one, some or all of the cranial devices.
27. A wearable cranial device assembly according to aspect 26, wherein the display device is configured to automatically switch to display the data for a cranial device for which a potential injury or health issue has been identified.
28. A wearable cranial device assembly according to aspect 26, wherein the display device includes a separate storage associated with each cranial device, each storage device storing general user data, historical health data and historical physiological data for the wearer of the associated cranial device.
29. A wearable cranial device assembly according to aspect 18, wherein the display device includes a transmitter for transmitting data received from the cranial device to at least one additional display device.

## Claims

1. A remote device for biometrically monitoring a subject for potential injury, the subject wearing a wearable cranial device, the remote device comprising:
a receiver configured to receive physiological data from at least one physiological sensor, impact data from at least one impact sensor, and environmental data from at least one environmental sensor, the at least one physiological sensor, the at least one impact sensor, and at least one environmental sensor associated with a wearable cranial device wearable by the subject; and
a processor configured to:
determine that at least one of the physiological data and the environmental data exceeds a corresponding threshold if the impact data indicates the at least one impact sensor has not detected an impact;
generate an alert based upon at least one of the physiological data and the environmental data exceeding the corresponding threshold if the impact data indicates the at least one impact sensor has not detected an impact, the alert usable to determine a potential injury to the subject;
determine, using the at least one impact sensor, the wearable cranial device experienced a current impact;
determine if an elevated alert should be generated based at least in part on a magnitude of the current impact and at least one additional factor, the at least one additional factor including at least one of the physiological data, the impact data and the environmental data; and
generate an elevated alert if a determination is made that an elevated alert should be generated.

2. The remote device of Claim 1, wherein one of the at least one additional factor is whether the wearable cranial device experienced at least one previous impact.

3. The remove device of Claim 2, wherein the processor is further configured to, if the wearable cranial device experienced no previous impact:
generate a low-level alert; and
revise a physiological data threshold to a more stringent threshold.

4. The remote device of Claim 1, wherein one of the at least one additional factor is whether the physiological data changed between an occurrence of at least one previous impact and an occurrence of a current impact.

5. The remote device of Claim 4, wherein the processor is further configured to generate the elevated alert if the wearable cranial device experienced at least one previous impact and if the physiological data changed between an occurrence of the at least one previous impact and an occurrence of the current impact.

6. The remote device of Claim 1, wherein the processor is further configured to:
correlate the physiological data and the environmental data; and
issue the alert based on the correlation of the physiological data and the environmental data even if the physiological data threshold and the environmental data threshold has not been reached.

7. The remote device of Claim 1, wherein the receiver is further configured to receive identification data of the wearable cranial device, wherein the alert provides an identification of the wearable device based on the received identification data.

8. The remote device of Claim 1, wherein the receiver is further configured to receive wearable device location data, wherein the alert provides a location of the wearable device based on the received wearable device location data.

9. The remote device of Claim 1, further including a display, wherein the receiver is further configured to receive impact magnitude and location data from the at least one impact sensor; and
the processor is further configured to cause the display of a graphical representation of a location and a magnitude of the current impact on a head of the subject using the magnitude and location data received from the at least one impact sensor.

10. The remote device of Claim 1, further including a display, wherein the processor is further configured to cause a display of telemetry from the wearable cranial device, the telemetry including any changes to at least one of the physiological data and the environmental data.

11. The remote device of Claim 1, wherein one of the at least one additional factor is a number of previous impacts to the wearable cranial device.

12. The remote device of Claim 1, wherein one of the at least one additional factor is a time frame between occurrences of previous impacts to the wearable cranial device.

13. The remote device of Claim 1, wherein the physiological data from at least one physiological sensor, the impact data from at least one impact sensor, and the environmental data from at least one environmental sensor are received from a plurality of wearable cranial devices, the remote device further comprising a transmission system configured to transmit the alert to a secondary remote device if the alert corresponds to the wearable cranial device associated with the secondary remote device.

14. A method for a remote device for biometrically monitoring a subject for potential injury, the method comprising:
receiving physiological data from at least one physiological sensor, impact data from at least one impact sensor, and environmental data from at least one environmental sensor, the at least one physiological sensor, the at least one impact sensor, and at least one environmental sensor associated with a wearable cranial device wearable by the subject;
determining that at least one of the physiological data and the environmental data exceeds a corresponding threshold if the impact data indicates the at least one impact sensor has not detected an impact;
generating an alert based upon at least one of the physiological data and the environmental data exceeding the corresponding threshold if the impact data indicates the at least one impact sensor has not detected an impact, the alert usable to determine a potential injury to the subject;
determining, using the at least one impact sensor, that the wearable cranial device experienced a current impact;
determining if an elevated alert should be generated based at least in part on a magnitude of the current impact and at least one additional factor, the at least one additional factor including at least one of the physiological data, the impact data and the environmental data; and
generating an elevated alert if a determination is made that an elevated alert should be generated.

15. The method of Claim 14, wherein the at least one additional factor includes one of:
whether the physiological data changed between an occurrence of at least one previous impact and an occurrence of a current impact;
whether the wearable cranial device experienced at least one previous impact;
a number of previous impacts to the wearable cranial device; and
a time frame between occurrences of previous impacts to the wearable cranial device.
